(19) Europäisches Patentamt
European Patent Office
Office européen des brevets

(11) Veröffentlichungsnummer: **0 237 978 A2**

# (12) EUROPÄISCHE PATENTANMELDUNG

(21) Anmeldenummer: **87103672.9**

(22) Anmeldetag: **13.03.87**

(51) Int. Cl.[4]: **C07C 35/06**, C07C 29/40, C11D 3/20, C11D 3/50

(30) Priorität: **21.03.86 DE 3609524**

(43) Veröffentlichungstag der Anmeldung: **23.09.87 Patentblatt 87/39**

(84) Benannte Vertragsstaaten: **BE CH DE ES FR GB LI NL**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Schaper, Ulf-Armin, Dr.**
**Randstrasse 18 a**
**D-4150 Krefeld(DE)**
Erfinder: **Bruns, Klaus, Prof. Dr.**
**Notburgaweg 6**
**D-4150 Krefeld-Traar(DE)**
Erfinder: **Blösl, Siegfried, Dr.**
**Isarstrasse 4**
**D-7070 Schwäbisch-Gmünd(DE)**
Erfinder: **Streschnak, Benno, Dr.**
**Schönwasserstrasse 259**
**D-4150 Krefeld(DE)**

## (54) Isomere 1-Alkyl/Alkenyl-2,2,4(2,4,4)-trimethyl-cyclopentan-1-ole als Riechstoffe.

(57) Die Erfindung betrifft Isomere 1-Alkyl/Alkenyl-2,2,4(2,4,4)trimethyl-cyclopentan-1-ole der allgemeinen Formel I a/b

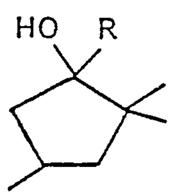

und

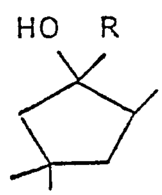

worin R eine Alkylgruppe mit 2 bis 5 Kohlenstoffatomen oder eine Alkenylgruppe mit 3 bis 5 Kohlenstoffatomen bedeutet.

EP 0 237 978 A2

Xerox Copy Centre

# Isomere 1-Alkyl/Alkenyl-2,2,4(2,4,4)-trimethyl-cyclopentan-1-ole als Riechstoffe

Die Erfindung betrifft isomere 1-Alkyl/Alkenyl-2,2,4(2,4,4)-trimethyl-cyclopentan-1-ole, ein Verfahren zu deren Herstellung sowie die Verwendung derselben als Riechstoffe in aktivchlorhaltigen Mischungen.

In vielen Verbrauchsgütern, beispielsweise in Körperpflegemitteln oder Wäscheweichspülern, sind Probleme hinsichtlich der Stabilität der verwendeten Riechstoffe unbekannt. Im Gegensatz dazu zerstören aktivchlorhaltige Mischungen wie beispielsweise Maschinengeschirrspülmittel oder Scheuermittel, die meisten Riechstoffe, so daß eine Parfümierung in den gewünschten Duftrichtungen nicht mehr möglich ist (siehe zum Beispiel in J.S. Jellinek: "Parfümieren von Produkten", S. 100/101, Dr. Alfred Hüthig Verlag Heidelberg, 1976). Zusätzlich ist in vielen Fällen die Zerstörung der Riechstoffe mit einer unerwünscht hohen Reduzierung des Aktivchlorgehaltes verbunden.

Die Aufgabe der Erfindung bestand daher in der Bereitstellung von Riechstoffen, die in aktivchlorhaltigen Mischungen stabil sind.

Überraschenderweise wurde gefunden, daß bei Verwendung isomerer 1-Alkyl/Alkenyl-2,2,4(2,4,4)-trimethyl-cyclopentan-1-ole der allgemeinen Formel I a/b

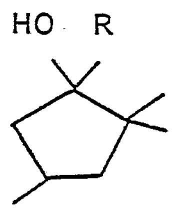

und

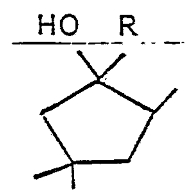

worin R eine Alkylgruppe mit 1 bis 5 Kohlenstoffatomen oder eine Alkenylgruppe mit 2 bis 5 Kohlenstoffatomen bedeutet, als Riechstoffe in aktivchlorhaltigen Mischungen keine Stabilitäts-probleme auftreten.

Gegenstand der Erfindung sind isomere 1-Alkyl/Alkenyl-2,2,4-(2,4,4)-trimethyl-cyclopentan-1-ole der allgemeinen Formeln I a/b

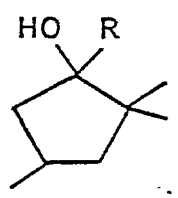

und

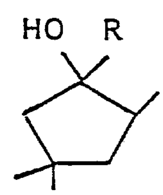

worin R eine Alkylgruppe mit 2 bis 5 Kohlenstoffatomen oder eine Alkenylgruppe mit 3 bis 5 Kohlenstoffatomen bedeutet.

Isomere Cyclopentan-1-ole der allgemeinen Formel I a/b, in der R für die Ethyl-, n-Butyl-oder Allylgruppe steht, sind besonders bevorzugt.

Ein weiterer Gegenstand der Erfindung ist ein Verfahren zur Herstellung isomerer Cyclopentan-1-ole der allgemeinen Formel I a/b durch Umsetzung isomerer 2,2,4(2,4,4)-Trimethyl-cyclopentanone der allgemeinen Formel II a/b

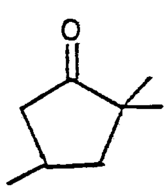

und

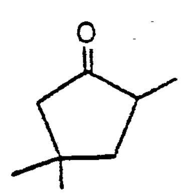

mit Grignard-Verbindungen R-MgX, worin R für $C_2$-$C_5$-Alkyl oder $C_3$-$C_5$-Alkenyl, vorzugsweise für Ethyl, n-Butyl oder Allyl, und X für ein Halogenatom steht, in Ether bei etwa 36 °C. Die gebildeten Salze werden durch anschließende Hydrolyse in die isomeren Cyclopentan-1-ole der allgemeinen Formel I a/b überführt.

Gegenstand der Erfindung ist ferner die Verwendung isomerer 1-Alkyl/Alkenyl-2,2,4(2,4,4)-trimethyl-cyclopentan-1-ole der allgemeinen Formel I a/b

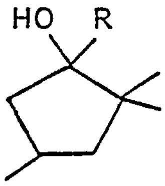

und

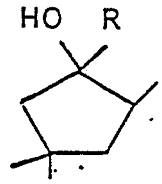

worin R eine Alkylgruppe mit 1 bis 5 Kohlenstoffatomen oder eine Alkenylgruppe mit 2 bis 5 Kohlenstoffatomen bedeutet, als Riechstoffe in aktivchlorhaltigen Mischungen.

Isomere Cyclopentan-1-ole der allgemeinen Formel I a/b, worin R Methyl, Ethyl, n-Butyl, Vinyl oder Allyl bedeutet, werden bevorzugt als Riechstoffe in aktivchlorhaltigen Mischungen verwendet.

Die Cyclopentan-1-ole 1,cis-2,4,4-und 1,trans-2,4,4-Tetramethyl-cyclopentan-1-ol sowie das isomere 1-Vinyl-2-2,4(2,4,4)-trimethyl-cyclopentan-1-ol sind bereits in der Literatur beschrieben worden. Die beiden Methylverbindungen wurden von Min-Hon Rei (J.Org. Chem. 43, 2173 (1978) ) während seiner Untersuchungen über die Reaktionen Methyl-substituierter Cyclopentanone mit Lithiumaluminiumhydrid und Methyllithium durch Umsetzung von 2,4,4-Trimethylcyclopentan-1-on mit Methyllithium in Ether bei 0 °C dargestellt. In EP-PS 21 356 wird das Vinyl-substituierte Cyclopentanol als Vorstufe zur Herstellung von 4(5)-Acetyl-7,7,9(7,9,9)trimethyl-bicyclo 4.3.0 non-1-en beschrieben. Die Herstellung erfolgt durch Umsetzung von 2,2,4(2,4,4)-Trimethylcyclopentanon mit Vinylmagnesiumbromid in Tetrahydrofuran bei etwa 66 °C in 12 Stunden.

In keiner der beiden Literaturstellen werden jedoch Angaben über den Geruch der erhaltenen Cyclopentanole sowie deren Stabilität gegenüber aktivchlorhaltigen Mischungen gemacht.

Die Herstellung der isomeren Cyclopentanole der allgemeinen Formel I a/b

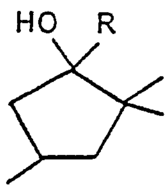

und

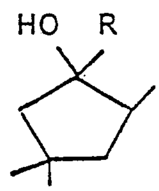

worin R eine Alkylgruppe mit 1 bis 5 Kohlenstoffatomen oder eine Alkenylgruppe mit 2 bis 5 Kohlenstoffatomen bedeutet, erfolgt nach einem an sich bekannten Syntheseverfahren der organischen Chemie: isomeres 2,2,4(2,4,4)-Trimethylcyclopentanon wird mit etwa 10 bis 15 Mol%igem Überschuß (bezogen auf eingesetztes Alkylhalogenid) des entsprechenden Alkyl-oder Alkenyl-magnesiumhalogenids in Ether bei etwa 36 °C umgesetzt. Anschließend wird die Reaktionsmischung auf etwa 0 °C abgekühlt und hydrolysiert.

Die isomeren Cyclopentan-1-ole der allgemeinen Formel I a/b

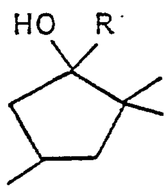

und

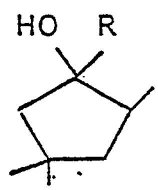

worin R eine Alkylgruppe mit 1 bis 5 Kohlenstoffatomen oder eine Alkenylgruppe mit 2 bis 5 Kohlenstoffatomen, vorzugsweise Methyl, Ethyl, n-Butyl, Vinyl oder Allyl, bedeutet, wurden einem Lagertest unterworfen. Dabei wurde überraschenderweise gefunden, daß selbst nach einem Jahr bei Raumtemperatur in flüssigen und festen aktivchlorhaltigen Mischungen keine Geruchsänderungen auftraten.

## Beispiele

### Allgemeines Verfahren zur Herstellung von 1-Alkyl/Alkenyl-2,2,4(2,4,4)trimethyl-cyclopentan-1-ole

In die ausgeheizte Apparatur werden zu 0,5 Mol Magnesium-Spänen in 50 ml wasserfreiem Ether 20 ml einer Lösung aus 0,5 Mol Alkylhalogenid in 150 ml Ether getropft. Sobald sich das Grignard-Reagens zu bilden beginnt, wird die restliche Alkylhalogenid-Lösung so schnell zugefügt, daß der Ether schwach siedet. Nach beendeter Zugabe wird noch etwa 1/2 Stunde unter Rückfluß erhitzt.

Zu der abgekühlten Grignard-Lösung werden unter Rühren 0,45 Mol 2,2,4(2,4,4)-Trimethylcyclopentanon (Isomerenverhältnis 2,2,4 : 2,4,4 = 61 : 39) in 100 ml Ether getropft und etwa 1/2 Stunde unter Rückfluß gerührt.

Zur Aufarbeitung wird die Reaktionsmischung auf 0 °C abgekühlt und vorsichtig in eine kalte, gesättigte $NH_4Cl$-Lösung (etwa 300 ml) gegossen. Anschließend wird die Etherphase abgetrennt und die wäßrige Phase 2mal mit je 80 ml Ether extrahiert. Die vereinigten etherischen Phasen werden mit gesättigter Natriumhydrogencarbonat-und Natriumchloridlösung gewaschen. Nach Trocknen mit Natriumsulfat und Abdampfen des Lösungsmittels wird der Rückstand i.Vak. destilliert.

1,2,2,4(1,2,4,4)-Tetramethyl-cyclopentan-1-ol
Kp 90 °C/70 mbar, $n_D^{20}$ = 1,4476
Geruch: camphrig, borneolig, Rosmarin-Note.

1-Ethyl-2,2,4(2,4,4)-trimethyl-cyclopentan-1-ol
Kp 64 °C/12 mbar, $n_D^{20}$ = 1,4520
Geruch: camphrig, holzig, Pyrethrum-, Anthoxan-Note

1-n-Butyl-2,2,4(2,4,4)-trimethyl-cyclopentan-1-ol
Kp 80 °C/39 mbar, $n_D^{20}$ = 1,4527
Geruch: erdig, holzig, Geosmin-Note

1-Vinyl-2,2,4(2,4,4)-trimethyl-cyclopentan-1-ol
Kp 67 °C/19 mbar, $n_D^{20}$ = 1,4601
Geruch: Campher-, Verdol-Note

1-Allyl-2,2,4(2,4,4)-trimethyl-cyclopentan-1-ol
Kp 114 °C/117 mbar, $n_D^{20}$ = 1,4631
Geruch: Patchone-, Kräuternote.

### Bouquet für flüssige und feste aktivchlorhaltige Mischungen

30 Gew.-Teile Campher synth.
20 Gew.-Teile Moschus Xylol
100 Gew.-Teile 4-(4-Methyl-3-penten-1-yl)-3-cyclohexen-1-carbaldehyd
140 Gew.-Teile Isobornylacetat
100 Gew.-Teile Eucalyptol
10 Gew.-Teile Nelkenblätteröl
200 Gew.-Teile 1,2,2,4(1,2,4,4)-Tetramethyl-cyclopentan-1-ol
180 Gew.-Teile Dihydromyrcenol
20 Gew.-Teile 2,6-Dimethylheptan-2-ol
100 Gew.-Teile Rosmarinöl span.
100 Gew.-Teile Dipropylenglycol

**Ansprüche**

1. Isomere 1-Alkyl/Alkenyl-2,2,4(2,4,4)trimethyl-cyclopentan-1-ole der allgemeinen Formel I a/b

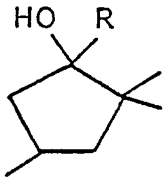

und

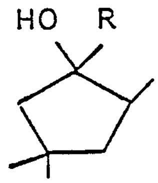

worin R eine Alkylgruppe mit 2 bis 5 Kohlenstoffatomen oder eine Alkenylgruppe mit 3 bis 5 Kohlenstoffatomen bedeutet.

2. Isomere Cyclopentan-1-ole der allgemeinen Formel I a/b nach Anspruch 1, dadurch gekennzeichnet, daß R Ethyl, n-Butyl oder Allyl bedeutet.

3. Verfahren zur Herstellung isomerer Cyclopentan-1-ole nach den Ansprüchen 1 und 2, dadurch gekennzeichnet, daß isomeres 2,2,4(2,4,4)-Trimethylcyclopentanon der allgemeinen Formel II a/b

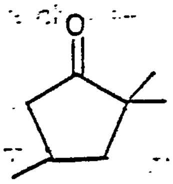

und

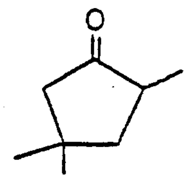

mit Grignard-Verbindungen R-MgX, worin R für $C_2$-$C_5$-Alkyl oder $C_3$-$C_5$-Alkenyl, vorzugsweise für Ethyl, n-Butyl oder Allyl, und X für ein Halogenatom steht, in Ether bei etwa 36 °C umgesetzt wird und die gebildeten Salze durch anschließende Hydrolyse in die isomeren Cyclopentan-1-ole der allgemeinen Formel I a/b überführt werden.

4. Verwendung isomerer 1-Alkyl/Alkenyl-2,2,4(2,4,4)trimethyl-cyclopentan-1-ole der allgemeinen Formel I a/b

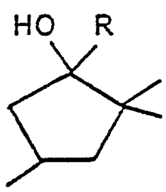

und

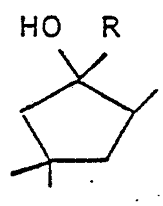

worin R eine Alkylgruppe mit 1 bis 5 Kohlenstoffatomen oder eine Alkenylgruppe mit 2 bis 5 Kohlenstoffatomen bedeutet, als Riechstoffe in aktivchlorhaltigen Mischungen.

5. Verwendung isomerer Cyclopentan-1-ole nach Anspruch 4, dadurch gekennzeichnet, daß R Methyl, Ethyl, n-Butyl, Vinyl oder Allyl bedeutet.